# EUROPEAN PATENT SPECIFICATION

(11) **EP 2 926 726 B1**
(45) Date of publication and mention of the grant of the patent: **08.05.2019**
(21) Application number: 15161649.7
(22) Date of filing: 30.03.2015
(51) Int. Cl.: A61B 5/024, A61B 5/00

(54) **HEART RATE MONITOR AND STRAP**
HERZFREQUENZÜBERWACHUNG UND GURT
MONITEUR DE FRÉQUENCE CARDIAQUE ET SANGLE

(30) Priority: 28.03.2014 GB 201405673
(43) Date of publication of application: 07.10.2015
(73) Proprietor: TomTom International B.V., 1011 AC Amsterdam (NL)
(72) Inventor: Magniez, Clement Albert Anne, 1011 AC Amsterdam (NL); Jackson, Stephen, 1011 AC Amsterdam (NL)
(74) Representative: Dobson, Adrian

(56) References cited:
- WO-A1-2014/070560
- CA-A1- 2 883 852
- GB-A- 2 494 622
- US-A1- 2012 271 121

## Description

### Field of the Invention

This invention relates to heart rate monitors, and more specifically optical heart rate (OHR) sensors. Furthermore the invention relates to a wearable heart rate monitor comprising an OHR sensor and to the mounting of such a sensor, which in preferred embodiments takes the form of a strap. The OHR sensor may be provided as a module that can be removably mounted to the strap. The invention further relates to a strap for mounting a device such as a heart rate monitor to a limb of a user. Illustrative embodiments of the invention relate to devices for monitoring athletic performance, e.g. that can be worn during an exercise activity (running, cycling, swimming, hiking, skiing, weightlifting, etc.), which can track, display and record the heart rate of the user at particular moments during a workout.

### Background of the Invention

Conventional heart rate monitors typically take the form of a sensor mounted to a chest strap. The capacitive sensor detects the heart's electrical activity through the skin. This requires the chest strap to mount the sensor close to the heart and to hold it firmly against the skin. A user wears the chest strap and a corresponding device, such as a wrist watch, which is wirelessly linked to the heart rate sensor to receive and display heart rate information. Alternatively the sensor mounted to the chest strap might be linked to a non-wearable display device such as a mobile phone or a treadmill controller.

Alternatively, some heart rate monitors can be worn on the wrist rather than using a chest strap. These pulse monitors require a user to touch a finger against a pad to provide a pulse rate. However this type of monitor requires the user to stop exercising to take the pulse reading and tends to be less accurate than the chest strap monitors.

More recently, strapless heart rate monitors have taken the form of wrist-worn devices that use optical sensing of the volume of blood under the skin. WO 2013/042070 A1 discloses an optical heart rate monitor that includes a housing in the form of a watch for wearing on the wrist or arm of a user. The monitor comprises an LED arranged to emit light into the skin of a user, where it is partially absorbed by the underlying blood vessels, and a photodetector arranged to sense light reflected back through the skin. The monitor processes the sensor signals and determines a pulse and/or heart rate for display.

Optical heart rate monitors must be mounted in stable contact with the skin during the use to avoid motion artefacts and ensure reliable measurements. Furthermore, ambient light artefacts can reduce measurement quality, for example when a user moves between locations of different ambient lighting, e.g. between shadow and direct sunlight. In WO 2013/042070 A1 an optical high-pass filter is used when detecting the reflected light signals so as to filter out ambient infrared light.

It is desired, in at least embodiments of the present invention, to provide an improved heart rate monitor and strap for mounting a heart rate monitor or similar device.

### Summary of the Invention

A first aspect of the present invention provides a heart rate monitor comprising an optical heart rate (OHR) sensor and a strap for mounting the sensor to a limb of a user, the OHR sensor comprising a sensing unit including at least one light emitter arranged to emit light into the skin of a user and a photodetector arranged to sense light reflected through the skin of the user, the sensing unit being arranged substantially centrally in the strap and the strap comprising compressible edge portions that run along opposite sides of the sensing unit such that, when the strap is mounted to a limb of a user, the edge portions are compressed against the skin to prevent or reduce the ingress of ambient light to the sensing unit.

According to the present invention, the compressible edge portions of the strap act to mechanically prevent ambient light from reaching the sensing unit where it might interfere with the measurement quality of the OHR sensor. The compressibility of the edge portions means that that they can react to the strap flexing during wear and retain a seal against the skin. As a result, the photodetector may not need to include an optical filter to remove light artefacts. Furthermore, it is believed that the compressible edge portions may also have the dual effect of preventing or reducing motion artefacts by helping to hold the sensing unit in a fixed position against the skin even when a user is moving during exercise. The present invention is therefore able to overcome the problems outlined above.

It will be appreciated that the compressible edge portions run along an edge of the strap, which is elongate by nature, and hence the edge portions will be understood to run along a longitudinal direction of the strap. Other edges of the strap, e.g. at its distal ends, are typically used to fasten the strap around a limb, e.g. being connected to a buckle or other fastener. The strap may be dimensioned to fit around a limb in the form of an arm, especially a wrist, and/or a limb in the form of a leg, for example an ankle. Miniature forms of the heart rate monitor may even comprise a strap sized for smaller limbs such as fingers or toes.

Preferably the sensing unit protrudes from a lower surface of the OHR sensor, which can ensure that the sensing unit contacts the skin of a wearer even if other parts of the sensor do not. This is particularly suitable when the strap is used to hold the sensing unit against the surface of a curved limb such as an arm or leg. The compressible edge portions of the strap may have a depth that is slightly greater than that of the sensing unit protruding from the lower surface, such that, when the strap is mounted to a limb of a user, the edge portions are compressed against the skin to a depth approximately equal to that that of the sensing unit. However, it has been found that one of the most important factors for accurate readings from the OHR sensor is for the sensing unit to be pressed against the skin in a uniform manner. If the edge portions are deeper than the sensing unit and the strap is not tightened to compress the edge portions far enough then they may detrimentally act to lift the sensing unit away from the surface of the skin. Accordingly it is preferable that each compressible edge portion has, in its uncompressed state, a depth equal to that of the sensing unit protruding from the lower surface or less than that of the sensing unit protruding from the lower surface.

The compressible edge portions of the strap do not need to contact the OHR sensor in order to prevent light from reaching the sensing unit. For example, the compressible edge portions might be spaced away from the OHR sensor so that the sensor does not interfere with the edge portions forming a seal against the wearer's skin. However in at least some embodiments it may be convenient that each compressible edge portion forms a flange extending over an edge of the OHR sensor. The compressible edge portions may thereby assist in holding the OHR sensor in position in the strap. This can be help to ensure that the OHR sensor is held in a stationary position in the strap and can not flex or move around even if the monitor is worn during strenuous exercise.

In at least some embodiments, the OHR sensor may be permanently mounted by the strap. For example, the OHR sensor may be integrated with the strap. However, in various embodiments of the present invention, the OHR sensor may be removably mounted by the strap. For example, the strap may comprise a central mount to which the OHR sensor is removably connected. This can allow the OHR sensor to be repeatedly engaged and disengaged from the strap, for example so that a user can dock the sensor to allow for the transfer of power and/or data, e.g. using a docking station connected to a computer. In addition, or alternatively, the same strap may be used interchangeably to mount different sensors or other units, such as a watch module including location determining means, e.g. a global navigation satellite system (GNSS) receiver, such as GPS and/or GLONASS.

As is mentioned above, the compressible edge portions of the strap can provide a superior fit to a user's limb regardless of whether the strap is used to mount an OHR sensor or another device. Accordingly the strap is considered to be novel and inventive in its own right.

A second aspect of the present invention provides a strap for mounting a device to a limb of a user, the strap comprising a central mount to hold the device against the skin of a user and compressible edge portions that run along opposite sides of the central mount such that, when the strap is mounted to a limb of a user, the edge portions are compressed against the skin to assist in holding the device in a fixed position.

Some strap-mounted devices are sensitive to motion artefacts resulting from a loose fit against a wearer's skin or other surface, e.g. the handlebar of a bicycle. Alternatively, or in addition, some strap-mounted devices may cause chafing or discomfort to a wearer if allowed to move against the skin during wear. This aspect of the invention may therefore provide a strap for mounting a range of different devices.

There will now be described some preferred features of a strap in embodiments according to either aspect of the present invention.

As is described above, the strap comprises compressible edge portions that run along opposite sides of the central mount or centrally mounted sensing unit. The central mount provided by the strap may comprise a physical connector for the OHR sensor or other device, for example a mechanical and/or magnetic connection system. In a preferred set of embodiments the central mount comprises an aperture in the strap, e.g. into which a device can be inserted. The device may comprise one or more projections and/or recesses for releasably engaging with corresponding features of the aperture.

The compressible edge portions may be the same, or a different, material to the rest of the strap. For example, a first material may be chosen for the compressible edge portions for its sealing properties while one or more second materials may be used to make the strap, e.g. taking into consideration flexibility, durability, water resistance, etc. Thus in some embodiments the compressible edge portions are made of a first material and the remainder of the strap is made from one or more second materials. In some other embodiments the compressible edge portions and the remainder of the strap may be made from the same first material. For example, the compressible edge portions may be integrally formed with the strap. This may provide for ease of manufacture, e.g. moulding, and remove the risk of the edge portions delaminating from the strap during wear.

The first material used at least for the compressible edge portions may be any suitable compressible material, for example a composite, foam, rubber or plastics material. Preferably the first material undergoes elastic compression, i.e. such that it substantially returns to its original form when no longer compressed. To prolong the lifetime of the strap, the first material may preferably be able to undergo many cycles of compression without loss of performance. In some embodiments the first material comprises a thermoplastic and/or elastomer. A suitable thermoplastic may be polyurethane. The elastomer may be thermoplastic or thermoset (i.e. requiring vulcanization), for example natural or artificial rubber, e.g. silicone rubber. The first material may comprise a combination of thermoplastic and elastomer materials. In a preferred set of embodiments the first material is a thermoplastic silicone vulcanizate. Such a material combines a thermoplastic, e.g. thermoplastic polyurethane (TPU), with silicone rubber, for example a dispersion of silicone internal phase produced by dynamic vulcanization or crosslinking of silicone polymers within a thermoplastic phase, resulting in a stable droplet-type morphology. Such a material can combine the silky feel and superior chemical and heat resistance properties of silicone rubber with the benefits of excellent abrasion resistance of a thermoplastic such as polyurethane. Advantageously this material may be fully processable and recyclable as a thermoplastic. A suitable thermoplastic silicone vulcanizate material is sold as TPSiV® by Multibase Inc., of the Dow Corning Corporation. Such a material may be characterised, in terms of its compressibility, as having a compression set at 23ºC, measured according to ISO 815, in the range of 20%-40%. In addition, or alternatively, such a material may be characterised, in terms of its compressibility, as having a compression set at 70ºC, measured according to ISO 815, in the range of 70%-90%. Thus in at least some embodiments the first material may be material chosen to have a compression set at 23ºC, measured according to ISO 815, in the range of 20%-40%, and preferably in the range of 25%-35%.

According to a third aspect of the present invention there is provided a strap for mounting a device to a limb of a user, the strap comprising a central mount to hold the device against the skin of a user and compressible edge portions that run along opposite sides of the central mount, the compressible edge portions made of a material having a compression set at 23ºC, measured according to ISO 815, in the range of 20%-40%. Preferably the material has a compression set at 23ºC, measured according to ISO 815, in the range of 25%-35%. The material of the compressible edge portions may comprise a thermoplastic and/or elastomer, as described above, for example a thermoplastic silicone vulcanizate.

In relation to the second and third aspects of the present invention, any of the embodiments described above may provide a strap for mounting a device comprising one or more of: a watch, a GPS watch, a speed sensor, a cadence sensor, an accelerometer, a gyroscope, an altimeter, a pressure sensor (e.g. diving depth gauge), an electronic compass, a sensor display unit (for example linked wirelessly to one or more body-worn sensors), a wireless communication hub (for example capable of transmitting signals from one or more body-worn sensors), and a heart rate monitor. Preferably the device comprises an optical heart rate (OHR) sensor and, in use, the edge portions are compressed against the skin to prevent or reduce the ingress of ambient light to the OHR sensor. Thus in preferred embodiments the strap is useable to mount an OHR sensor. As is described above, the OHR sensor may comprise a sensing unit including at least one light emitter arranged to emit light into the skin of a user and a photodetector arranged to sense light reflected through the skin of a user.

The OHR sensor or other device is preferably configured as a module housed by a single integral casing, and which is preferably a sealed module being water resistant to allow the device to be used for wet weather outdoor exercise and for swimming. In this configuration, the device can be removably mounted, i.e. repeatedly engaged and disengaged, to a plurality of different docking solutions, including the strap. When the device is not mounted by the strap, it can also be placed in a dock that is desired to be kept in the user's home, e.g. for allowing the transfer of power and/or data to the electrical components.

There will now be described some preferred features of an OHR sensor applicable to these embodiments, and to embodiments according to the first aspect of the present invention.

The OHR sensor may comprise a processor and a battery. The processor may be arranged to analyse light signals received, e.g. at the photodetector, for the purposes of display and/or transmission. The OHR sensor may comprise a memory connected to the processor. This means that HR data can be stored by the device and downloaded later. The OHR sensor may comprise an input/output (I/O) device for transferring data to and from the device and for providing power to recharge the battery. In some examples the OHR sensor may simply act as a sensor hub, collecting and/or transmitting heart rate data for display by another device. This may allow the OHR sensor to be minimised in size.

In some examples the processor is preferably connected to a user interface comprising a display screen, such as an LCD display. The display screen may visually display heart rate (HR) data such as one or more of: current HR (bpm), average HR (bpm), maximum HR, minimum HR; current HR zone; a graphical representation of HR changes over time; and a graphical representation of the proportion of time spent in each of a plurality of HR zones over time. In addition, or alternatively, the OHR sensor may comprise an audio output, e.g. a beeper, and/or a haptic output, e.g. a vibrator, to alert a user to changes in the HR data. In various examples the OHR sensor may comprise a user interface including an input device, e.g. in the form of one or more push buttons. Such an input device can enable a user to change relevant functions, such as applicable HR zones.

In a preferred set of embodiments the OHR sensor may comprise a display housing that houses a display for displaying information to a user, and an input means for controlling the device, wherein the input means is spaced apart from the display housing. Accordingly the input means is preferably spaced apart from the display housing in a longitudinal direction of the strap. Preferably the sensing unit is arranged beneath the display housing, so that when the device is mounted in use the sensing unit presses down against the wearer's skin while the display is positioned on top, i.e. facing away from the limb, for ease of viewing. The display may be configured to display alphanumeric characters or icons such that upper parts of the characters or icons are arranged towards a first side of the display housing and the lower parts of the characters or icons are arranged towards a second, opposite side of the display housing. The input means is preferably spaced apart from the display housing in a direction from said first side to said second side. This configuration is useful when the user wears the display housing on the back of the wrist, as the user is easily able to view the display whilst controlling the device via the input means that is spaced apart from the display. Less preferably, the input means may be spaced apart from the display housing in a direction from said second side to said first side of the display housing. This configuration may be useful, for example, when the device is strapped to the handle bars of a bicycle or strapped to another vehicle, as the display can be directed towards the user whilst the user has easy access to the input means from above the device.

The input means of the user interface is configured to control the device in use. For example, the input means may control the functioning of the OHR sensor and may be used to navigate through a menu displayed on the device. The input means is therefore electrically connected to electronic components in the display housing. For example, a ribbon lead may extend between the display housing and the input means.

The input means preferably has a substantially planar surface arranged substantially parallel to and above an upper surface of the device. The input means is preferably configured to detect the movement of a user's finger across the substantially planar surface so as to provide an input to control the device, e.g. for navigating a menu displayed on the display of the device. The input means may therefore comprise a touchpad (or trackpad) utilising, for example, capacitive sensing to conductance sensing to translate the motion of a user's finger into an input to control the device. The touchpad may comprise a one-dimensional touchpad, and which is capable of sensing motion along a single axis, e.g. left-right or up-down. In other more preferred embodiments, the touchpad may comprise a two-dimensional touchpad, and which is capable of sensing motion in any direction, or at least left-right and up-down, on the plane defined by the substantially planar surface of the input means.

In other, albeit less preferred embodiments, the input means may comprise a pointing stick (or trackpad) that senses the force applied by a user's finger, e.g. by using a pair of resistive strain gauges, and translates it into an input to control the device.

Alternatively, the input means may comprise a two-way button having a continuous pressing surface and two actuators, the button being configured such that when a first portion of the pressing surface is depressed a first of said actuators is actuated so as to provide a first input to control the device, and when a second portion of the pressing surface is depressed a second of said actuators is actuated so as to provide a second input to control the device.

Alternatively, the input means may comprise a four-way button having a continuous pressing surface and four actuators, the button being configured such that when a first portion of the pressing surface is depressed a first of said actuators is actuated so as to provide a first input to control the device, when a second portion of the pressing surface is depressed a second of said actuators is actuated so as to provide a second input to control the device, when a third portion of the pressing surface is depressed a third of said actuators is actuated so as to provide a third input to control the device, and when a fourth portion of the pressing surface is depressed a fourth of said actuators is actuated so as to provide a fourth input to control the device. The pressing surface described herein is preferably a substantially planar surface parallel to and above a portion of a lower surface that contacts a user's limb in use. It is also contemplated that the input means may comprise any one or more mechanically actuated buttons or non-mechanically actuated buttons, such as virtual buttons on a touch-sensitive user interface, as desired.

The input means is preferably additionally, or alternatively, configured to be operated by being pressed in a direction that is substantially perpendicular to its substantially planar surface, in a direction from the upper surface towards the lower surface. This enables the user to use a single finger to operate the input means. The user does not need to use a second finger of the same hand to counter-balance the pressing of the input means, because the input means is arranged such that it is pressed against the wrist of the user wearing the strap.

In preferred embodiments in which the input means is configured to both detect the movement of a user's finger across the substantially planar surface and be pressed against the limb of the user, e.g. where in the input means comprises a depressible touch pad, the detected motion of the user's finger is used to navigate a menu for identifying a function to be selected, and the depression of the input means is used to select the identified function.

As discussed above, the input means preferably has a substantially planar surface arranged substantially parallel to and above a portion of the upper surface, and which in some embodiments comprises a pressing surface for controlling the device when pressed. In embodiments, the input means defines a chamber, e.g. beneath the planar (or pressing) surface, which (as discussed in more detail below) can be used to house one or more components of the device, rather than in the display housing.

In addition, or alternatively, the display housing preferably has a substantially planar display, such as an LCD display, arranged in a first plane and the input means preferably has a substantially planar (pressing) surface arranged in a second plane, wherein the first and second planes are at angles to each other. The dihedral (or torsion) angle between the two planes is preferably less the 90 degrees, and preferably between 20 and 70 degrees. In other words, the planes are imaginary intersecting planes and the sides of the planes facing the user's arm or wrist in use define an angle between them at the intersection, wherein the angle is preferably greater than 90 degrees and less than 180 degree. By providing the surfaces at an angle to each other, the user is enabled a good viewing angle of the display whilst operating the input means, when the device is mounted to a user's arm in use. As the input means is spaced away from the display housing, and hence away from the back of the user's wrist and around the side of the wrist in use, said angle also enables the input means to be orientated such that when it is pressed it is pressed against the user's wrist such that the user's wrist provides the counter-force necessary to balance the pressing force. The input means is therefore able to be operated with a single finger and without needing a second finger on the same hand to counter-balance the pressing force as in conventional devices having buttons around the periphery of the display housing.

In addition, or alternatively, the display housing is preferably physically connected to the input means by a connecting portion, wherein the connecting portion is curved or angled along the direction from the display housing to the input means. The connecting portion may be curved or angled such that when the display housing is arranged on the back of a user's wrist in use, the connecting portion curves or otherwise extends around the wrist such that the input means is located on the side of the user's wrist. The OHR sensor is preferably configured such that the input means is located on the medial side of the user's wrist when the display housing is located on the back of the wrist, the medial side being the side facing the user's body when the back of the hand is facing vertically upwards. In other less preferred embodiments the strap may form said connecting portion that connects the display housing portion and the input means. The strap may be flexible or formed from one or more pivotable sections so as to flex or pivot to form the curved or angled portion.

The display housing and the input means are preferably arranged in a single integral casing having a display housing portion and an input means portion separated by the connecting portion. The connecting portion is therefore preferably also part of the integral casing. In various embodiments the strap may comprise at least two apertures, and the display and input means may each project through a respective aperture in the strap.

The OHR sensor may provide the functions of a heart rate monitor alone, or in at least some embodiments the OHR sensor may be provided as part of a device providing additional functions, for example a sports or fitness watch. Thus in a set of embodiments the OHR sensor is integrated into a watch device. In such embodiments the OHR sensor may comprise a processor connected to means for tracking the location of a user as he or she moves from one location to another, e.g. by using information received from global navigation satellite signals, or by accessing and receiving information from WiFi access points or cellular communication networks. In preferred embodiments the watch device comprises a global navigation satellite system (GNSS) receiver, such as a GPS and/or GLONASS receiver, for receiving satellite signals indicating the position, and optionally speed, of the receiver (and thus user) at a particular point in time, and which receives updated information at regular intervals. As will be appreciated, this adds the functionality of tracking the location of the user as he or she moves from one location to another. The GNSS receiver may comprise an antenna, e.g. in the form of a patch antenna, for use in determining the location and movements of the user of the monitor. Where the input means defines a chamber, as mentioned above, his chamber may house the antenna so that it is separated from the display housing and therefore not located on the back of a user's wrist in use. Rather, the spacing of the chamber away from the display housing enables the antenna to be located on the side of the user's wrist in use. As such, when the device is being used for activities such as fast walking, jogging or running, the location sensor will naturally be directed, substantially vertically, upwards so as to be at the optimum angle to receive data from the satellites. Also, if the device is strapped to the handle bars of a bicycle then the display could be arranged to face the user, whilst the chamber, and thus antenna, are directed vertically upwards. The housing of the antenna in the chamber also provides the benefit of allowing the display housing of the device to have a thinner profile than is typically possible with conventional devices having location functionality, such as fitness watches, as the patch antenna no longer needs to be stacked on top of the battery.

Whether or not the OHR sensor is integrated with a fitness watch, it is contemplated that the device may comprise any one or more of the following components: a pressure sensor for measuring atmospheric pressure (for use in determining altitude and/or depth); a pulse sensor; a vibration device for indicating alerts to a user; an accelerometer; an electronic compass; a processor; or a wireless communications device, such as a Bluetooth module (e.g. capable of using the Bluetooth Low Energy (BLE) protocol).

In embodiments where the OHR sensor comprises a wireless communications device, this may be arranged to receive data from other sensors, such as a foot pod sensor or a speed/cadence sensor. The wireless communications device may even be arranged to communicate with an external heart rate monitor, for example a monitor mounted on a chest strap worn by the user. The input means may allow a user to switch between the internal OHR sensor and an external heart rate monitor. In addition, or alternatively, the wireless communications device may be arranged to transmit data to one or more external devices (e.g. a mobile phone device), especially if the OHR sensor does not include its own display.

The OHR sensor may comprise one or more electrical connectors for electrically connecting to a dock or cable for charging the battery and/or for transferring data to or from the processor. It is contemplated that any known electrical connector may be employed. In preferred embodiments, however, the one or more electrical connectors comprise electrical contacts, which may be flat and arranged substantially in line with, or recessed in, the lower surface of the case (for contacting with corresponding pogo pins in a docking system). The electrical contacts may be located in any portion of the lower surface of the case as desired, although in preferred embodiments the electrical contacts are located in the lower surface under the input means of the user interface, e.g. at the end of the lower surface that is distal from the display housing. This allows the user to see the display when the device is positioned in a docking system. By way of example, a system is described comprising a heart rate monitor as described above and a docking system for charging or exchanging data with the OHR sensor, wherein the docking system comprises docking electrical connectors for connecting with the electrical connectors on the OHR sensor in order to supply electrical charge and/or data to the electrical connectors of the watch and/or to receive data from the watch.

The dock preferably has a slot for receiving at least a portion of the device therein, and wherein the electrical connectors of the device and the docking connectors are arranged and configured to engage with each other within the slot.

The electrical connectors on the watch are preferably electrical contacts, which may be flat and may be arranged substantially in line with a surface of the OHR sensor so as not to catch against a wearer's skin in use. The electrical connectors in the dock may be resiliently biased connectors, such as pogo connectors, that are biased into engagement with the connectors on the monitor when the device is docked. However, it is contemplated that any of the well known means of electrical connectors may be employed on the monitor or in the dock.

Advantages of these embodiments are set out hereafter, and further details and features of each of these embodiments are defined in the accompanying dependent claims and elsewhere in the following detailed description.

### Brief Description of the Drawings

Various aspects of the teachings of the present invention, and arrangements embodying those teachings, will hereafter be described by way of illustrative example with reference to the accompanying drawings, in which:
Figures 1A to 1C show different views of a strap according to a preferred embodiment;
Figure 2 shows a perspective view of an optical heart rate (OHR) sensor module according to a preferred embodiment;
Figure 3 shows a perspective view of the sensor module mounted to the strap;
Figures 4A and 4B provide alternative views of the strap-mounted device, showing the OHR sensing unit on an underside of the sensor module; and
Figure 5 provides a system overview for a sensor module in a fitness monitoring ecosystem.
Like reference numerals are used for the like features throughout the drawings.

### Detailed Description of the Preferred Embodiments

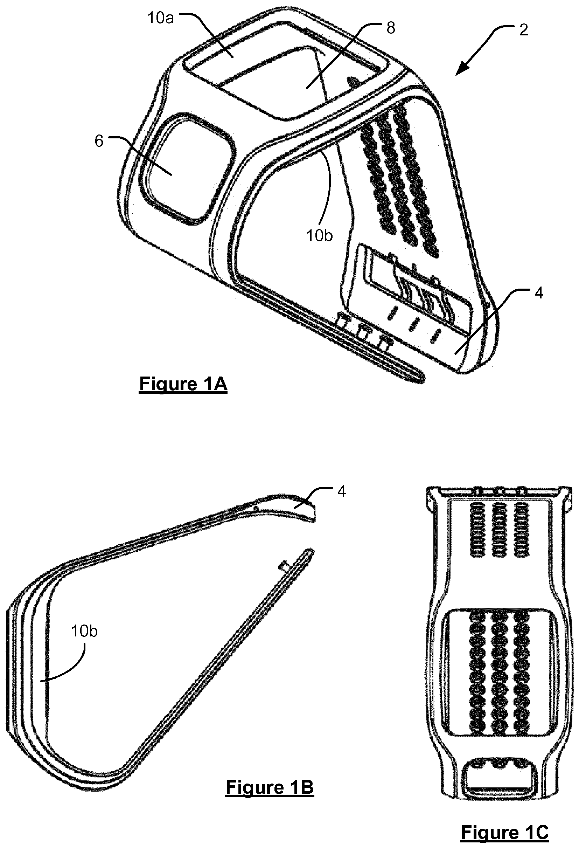

There is seen in Figures 1A to 1C a strap 2 for mounting a device to a limb of a user, in particular for mounting a device to the wrist. The strap 2 is generally flexible and comprises a buckle 4 at one end to enable it to be fastened around a wrist. It can be seen that, in this example, the strap 2 includes a central mounting aperture 6 in its upper plane and a further aperture 8 in a second plane that is angled away from the upper plane. These two apertures 6, 8 are arranged to receive the upwardly protruding parts of a curved device when it is connected to the strap. Of course the strap 2 may have any suitable shape, including any number of apertures, to match a particular device and enable connection to, and disconnection from, the strap 2. The strap 2 includes a pair of compressible edge portions 10a, 10b running along opposite sides of the central mounting aperture 6 and protruding downwardly from the upper surface of the strap 2. In this example the compressible edge portions 10a, 10b are seen to be made of the same material as the rest of the strap 2, but the remainder of the strap could of course be made of one or more different materials. At least the compressible edge portions 10a, 10b are made of a thermoplastic silicone vulcanizate or other material having similar compression and/or sealing properties. This means that, when the strap is worn on a user's wrist, the edge portions 10a, 10b can be compressed to form a seal against the user's skin and hold the strap 2 in place with any device mounted to the strap 2 being held firmly in a stationary position.

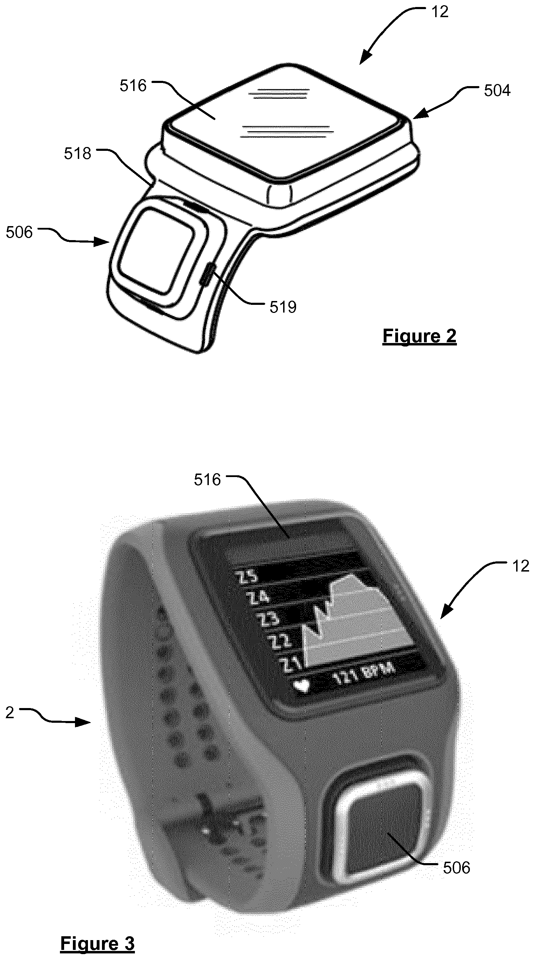

Figure 2 provides an example of a fitness monitoring device 12 that can be removably connected to the strap 2 seen in Figs. 1A to 1C. In some embodiments the device 12 could take the form of a fitness watch module, in particular a GNSS, e.g. GPS, watch module. In other embodiments the device 12 takes the form of an optical heart rate (OHR) sensor module, which may or may not include GNSS watch capabilities.

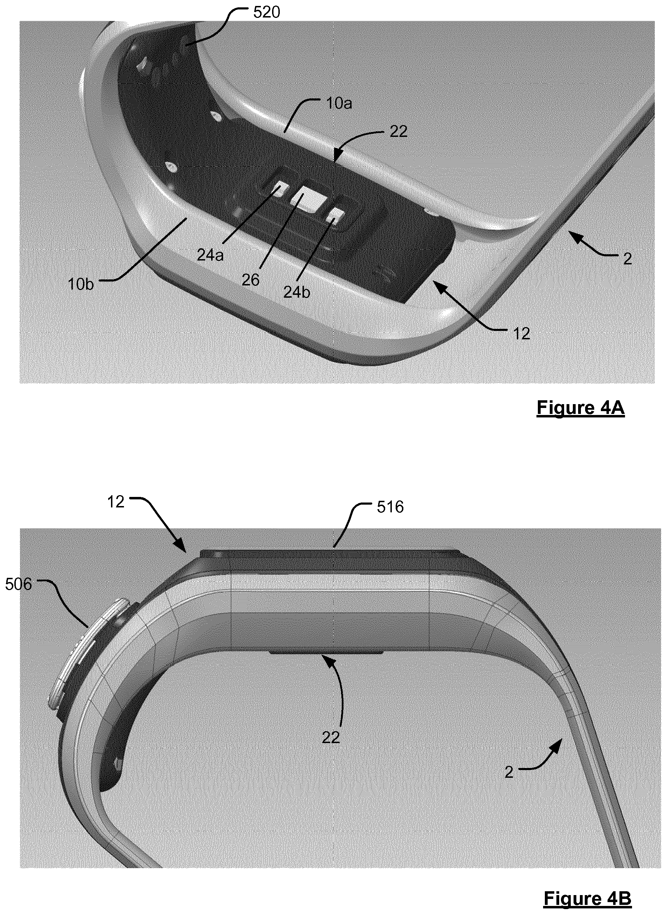

Figures 3, 4A and 4B show the OHR sensor module 12 connected to the strap 2.

It can be seen from Figures 2 to 4B that the sensor module 12 includes a user interface comprising a display housing 504 for a display, e.g. LCD 516, and a user input device, e.g. four-way button 506, which are spaced apart from each other by a connecting portion 518. This connecting portion 518 can be seen to curve away from the display housing 504 so as to curve around the side of a user's wrist then mounted to the strap 2. A user can therefore press the input device 506 from the side without interfering with view of the display 516. The sensor module 12 can be connected to the strap 2 by inserting the display housing 504 and user input device 506 through the respective apertures 6, 8. In the assembled heart rate monitor 20, the display 516 and the input device 506 are spaced apart in a longitudinal direction of the strap 2, with the display 516 facing upwardly and the input device 506 facing sideways. Cooperating projections and recesses on the sensor module 12 and the strap 2, e.g. the projections 519 seen in Figure 2, allow the module 12 to releasably engage and be maintained in position within the strap 2. Figure 3 shows an example of a graphical display relating to heart rate during use of the sensor module 12.

The location of the input device 506 being arranged on the curved portion 518 such that it sits against the side of the user's wrist in use has a number of important advantages. For example, this enables the user to interact with the sensor module 12 using only a single finger. More specifically, the user is able to push the pressing surface of the input device 506 with one finger because the user pushes the surface into the user's wrist. This is in contrast to conventional heart rate monitors or fitness watches wherein buttons are arranged around the peripheral edges of the device and the user must press the button with one finger and use a thumb on the other edge of the device to counter-balance the pressing force. In some examples, the input device 506 may define a chamber that houses one or more other components, such as a GPS patch antenna. By arranging a GPS antenna at the inner side of the wrist, the GPS antenna is in the optimum position for receiving GPS signals when worn by a user, as the inner side of the wrist will be directed substantially upwards during running or other exercise. As seen in Fig. 4B, for example, the plane defined by the substantially planar display 516 of the display housing 504 is arranged at an angle to the plane defined by the input device 506, the dihedral angle between the two planes being less than 90 degrees, and typically between 20 and 70 degrees.

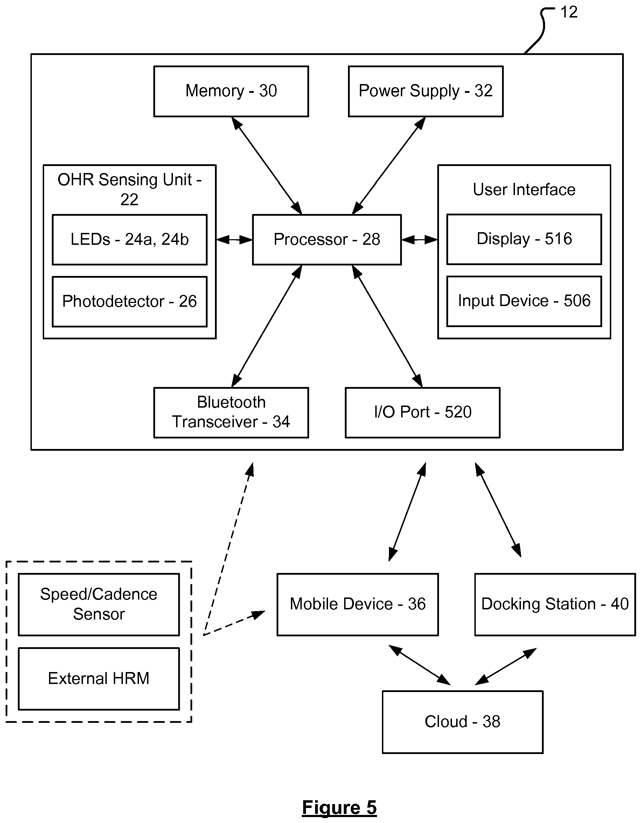

Fig. 4A shows a perspective view of the sensor module 12 from the underside. A distal end of the sensor module 12, at the same end as the input device 506, has an array of electrical connectors 520 exposed on its lower surface. These electrical connectors 520 may be used in order to electrically connect the sensor module 12 to a docking station in order to recharge a battery within the module 12 and/or to extract data from or input data to the module 12. As the electrical connectors 520 are arranged at a distal end of the sensor module 12, they are optimally arranged to be inserted into the slot of a docking device that has docking electrical connections for engaging the electrical connections 520. Alternatively, the electrical connectors 520 can be arranged to connect with a cable, e.g. a USB cable. It will be appreciated that removing the sensor module 12 from the strap 2 enables the sensor module 12 to be docked or connected to a cable more easily. The electrical connectors 520 may be used as an input/output port, as is described below with reference to Figure 5.

It can also be seen in Fig. 4A that the lower surface of the sensor module 12 has a sensing unit 22 protruding therefrom. The sensing unit 22 comprises a pair of light emitting diodes 24a, 24b (e.g. green LEDs), either side of a photodetector 26. When the sensor module 12 is mounted to a wrist by the strap 2, the sensing unit 22 sits on the back of the wrist in contact with the skin. It is also seen that the compressible edge portions 10a, 10b extend around the sides of the sensor module 12 to assist in holding it in place, and protrude in the same direction as the sensing unit 22. However, the compressible edge portions 10a, 10b do not protrude beyond the sensing unit 22, and preferably not even as far as the sensing unit 22, so that they do not interfere with the sensing unit 22 being pressed closely against the wearer's skin. The compressibility of the edge portions 10a, 10b means that they readily spread sideways when the module 12 is held against a user's wrist, forming a seal along opposite sides of the sensing unit 22 so as to prevent or reduce the ingress of ambient light to the photodetector 26.

Figure 5 provides an illustrative representation of some of the main electronic components in the sensor module 12, in block component form. It should be noted that the block diagram of Figure 5 is not exhaustive or inclusive of all components of the device, but merely representative of some example components in a typical sensor module 12. It can be seen that the optical heart rate (OHR) sensing unit 22, comprising the LEDs 24a, 24b and photodetector 26, is operatively connected to a processor 28 that can process data signals relating to pulse and heart rate. The processor 28 is connected to a memory 30 and a power supply, e.g. battery 32. The battery 32 may be recharged when the module 12 is docked using its I/O port, for example in the form of the electrical connections 520 described above. The same electrical connections 520 may also be used to transfer data to/from the processor. In addition to the I/O port, the sensor module 12 includes a wireless communications interface, such as a Bluetooth transceiver 34. The module's user interface includes the display 516 and input device 506 already described above. Of course other user interface components may be provided instead, or as well as, those seen in the figures.

The Bluetooth transceiver 34 optionally enables the sensor module 12 to wirelessly communicate with one or more other devices. For example, other devices that are body-worn or mounted nearby during exercise (e.g. mounted to a bike during cycling activities) may pair with the sensor module 12 to transmit additional data. The examples illustrated in Figure 5 are a speed/cadence sensor and/or an external heart rate monitor (HRM), for example a monitor mounted to a chest strap. The sensor module's user interface may allow a user to view such additional data on the wrist-mounted device.

The Bluetooth transceiver 34 enables the sensor module 12 to wirelessly communicate with a mobile device 36, for example a user's smart phone. The ecosystem may include an app downloaded onto the mobile device 36 to provide a dedicated interface for the sensor module 12. This may enable a user to track and save his or her workouts and share them via social media. The mobile device 36 may act as a wireless access point, enabling data to be transmitted wirelessly (via WiFi or a cellular connection) to the cloud 38 and for updates to be downloaded to the mobile device 36 and/or to the sensor module 12. In addition to such wireless communication, the I/O port enables the sensor module 12 to be physically connected to a docking station 40, for example connected to a desktop computer, so that data can be transferred periodically. The sensor module 12 may be removed from the strap 2 for ease of docking.

It will be appreciated that whilst various aspects and embodiments of the present invention have heretofore been described, the scope of the present invention is not limited to the particular arrangements set out herein and instead extends to encompass all arrangements, and modifications and alterations thereto, which fall within the scope of the appended claims.

For example, although the OHR sensor module has been described as having a display and/or input means, these are optional components. A suitable OHR sensor module may include a battery and a processor connected to one or more of: an optional display, an optional input device, a memory, a wireless transceiver, and an input/output device such as electrical contacts.

## Claims

1. A heart rate monitor, comprising:
an optical heart rate (OHR) sensor (12); and
a strap (2) for mounting the sensor to a limb of a user,
wherein the OHR sensor (12) comprises a sensing unit (22) including at least one light emitter (24a,24b) arranged to emit light into the skin of a user and a photodetector (26) arranged to sense light reflected through the skin of the user, the sensing unit (22) protruding from a lower surface of the OHR sensor (12), and the sensing unit (22) being arranged substantially centrally in the strap (2), and
wherein the strap (2) comprises compressible edge portions (10a,10b) that run along opposite sides of the sensing unit (22), wherein each compressible edge portion (10a, 10b) is configured to form a flange extending over an edge of the OHR sensor (12), and wherein the flange of each edge portion is configured such that, when the strap is mounted to a limb of a user, the flange of each edge portion is compressed against the skin to prevent or reduce the ingress of ambient light to the sensing unit.

2. The heart rate monitor of claim 1, wherein each compressible edge portion (10a,10b) has, in its uncompressed state, a depth equal to or less than that of the sensing unit (22) protruding from the lower surface of the OHR sensor (12).

3. The heart rate monitor of claim 1 or 2, wherein: (i) the compressible edge portions (10a,10b) are made of a first material and the remainder of the strap is made from one or more second materials; or (ii) the compressible edge portions (10a,10b) and the remainder of the strap are made from the same first material, and wherein the first material comprises a thermoplastic and/or elastomer.

4. The heart rate monitor of claim 3, wherein the first material is a thermoplastic silicone vulcanizate.

5. The heart rate monitor of claim 3 or 4, wherein the first material is configured to have a compression set at 23°C, in the range of 20%-40%.

6. The heart rate monitor of any preceding claim, wherein the sensing unit (22) comprises a pair of light emitting diodes (24a,24b), the photodetector (26) being arranged in between the pair of light emitting diodes.

7. The heart rate monitor of any preceding claim, wherein the OHR sensor (12) is removably mounted by the strap.

8. The heart rate monitor of any preceding claim, wherein the OHR sensor (12) comprises a display housing (504) that houses a display (516) for displaying information to a user, and an input device (506) for navigating through a menu displayed on the display (516) to control the functioning of the OHR sensor (12), wherein the input device (506) is spaced apart from the display housing (504).

9. The heart rate monitor of claim 8, wherein the input device (506) is spaced apart from the display housing (504) in a longitudinal direction of the strap.

10. The heart rate monitor of claim 8 or 9, wherein the display housing (504) has a substantially planar display (516) arranged in a first plane and the input device (506) has a substantially planar pressing surface arranged in a second plane, wherein the first and second planes are at angles to each other, wherein the angle between the first and second planes is less than 90 degrees, optionally between 20 and 70 degrees.

11. The heart rate monitor of any of claims 8 to 10, wherein the display housing (504) is physically connected to the input device (506) by a connecting portion (518), wherein the connecting portion is curved or angled along the direction from the display housing to the input device.

12. The heart rate monitor of claim 11, wherein the connecting portion (518) is curved or angled such that when the display housing (504) is arranged on the back of a user's wrist in use, the connecting portion (518) curves or otherwise extends around the wrist such that the input device (506) is located on the side of the user's wrist.

13. The heart rate monitor of claim any of claims 8 to 12, wherein the OHR sensor (12) has a lower surface for contacting the user's wrist in use and an upper, opposite surface for facing away from the user's wrist in use, and wherein the input device (506) has a substantially planar surface arranged substantially parallel to and above a portion of the lower surface, the input device (506) being configured to be operated by being pressed in a direction that is substantially perpendicular to its substantially planar surface, in a direction from the upper surface towards the lower surface.

14. The heart rate monitor of any of claims 8 to 13, wherein the strap (2) comprises at least two apertures (6,8), and the display housing (504) and input device (506) each project through a respective aperture in the strap.

## Patentansprüche

1. Herzfrequenzmonitor, der Folgendes umfasst:
einen optischen Herzfrequenz-(OHR)-Sensor (12); und
einen Riemen (2) zum Montieren des Sensors an einer Gliedmaße eines Benutzers,
wobei der OHR-Sensor (12) eine Sensoreinheit (22) mit wenigstens einem zum Emittieren von Licht in die Haut eines Benutzers ausgelegten Lichtemitter (24a, 24b) und einem zum Erfassen von durch die Haut des Benutzers reflektiertem Licht ausgelegten Fotodetektor (26) umfasst, wobei die Sensoreinheit (22) von einer Unterseite des OHR-Sensors (12) vorsteht und die Sensoreinheit (22) im Wesentlichen zentral in dem Riemen (2) angeordnet ist, und
wobei der Riemen (2) komprimierbare Randabschnitte (10a, 10b) umfasst, die entlang gegenüberliegenden Seiten der Sensoreinheit (22) verlaufen, wobei jeder komprimierbare Randabschnitt (10a, 10b) zum Bilden eines Flanschs konfiguriert ist, der sich über einen Rand des OHR-Sensors (12) erstreckt, und wobei der Flansch jedes Randabschnitts so konfiguriert ist, dass, wenn der Riemen an einer Gliedmaße eines Benutzers montiert ist, der Flansch jedes Randabschnitts gegen die Haut gedrückt wird, um das Eintreten von Umgebungslicht in die Sensoreinheit zu verhindern oder zu reduzieren.

2. Herzfrequenzmonitor nach Anspruch 1, wobei jeder komprimierbare Randabschnitt (10a, 10b) in seinem unkomprimierten Zustand eine Tiefe hat, die gleich oder kleiner ist als die der Sensoreinheit (22), die von der Unterseite des OHR-Sensors (12) vorsteht.

3. Herzfrequenzmonitor nach Anspruch 1 oder 2, wobei: (i) die komprimierbaren Randabschnitte (10a, 10b) aus einem ersten Material gefertigt sind und der Rest des Riemens aus einem oder mehreren zweiten Materialien gefertigt ist; oder (ii) die komprimierbaren Randabschnitte (10a, 10b) und der Rest des Riemens aus demselben ersten Material gefertigt sind, und wobei das erste Material einen Thermoplast und/oder ein Elastomer umfasst.

4. Herzfrequenzmonitor nach Anspruch 3, wobei das erste Material ein thermoplastisches Silikonvulkanisat ist.

5. Herzfrequenzmonitor nach Anspruch 3 oder 4, wobei das erste Material so konfiguriert ist, dass es einen Druckverformungsrest bei 23°C im Bereich von 20 % - 40 % hat.

6. Herzfrequenzmonitor nach einem vorherigen Anspruch, wobei die Sensoreinheit (22) ein Paar Leuchtdioden (24a, 24b) umfasst, wobei der Fotodetektor (26) zwischen dem Paar Leuchtdioden angeordnet ist.

7. Herzfrequenzmonitor nach einem vorherigen Anspruch, wobei der OHR-Sensor (12) durch den Riemen entfernbar montiert ist.

8. Herzfrequenzmonitor nach einem vorherigen Anspruch, wobei der OHR-Sensor (12) ein Anzeigegehäuse (504), das ein Display (516) aufnimmt, um einem Benutzer Informationen anzuzeigen, und ein Eingabegerät (506) zum Navigieren durch ein auf dem Display (516) angezeigtes Menü umfasst, zum Steuern des Betriebs des OHR-Sensors (12), wobei das Eingabegerät (506) von dem Anzeigegehäuse (504) beabstandet ist.

9. Herzfrequenzmonitor nach Anspruch 8, wobei das Eingabegerät (506) von dem Anzeigegehäuse (504) in einer Längsrichtung des Riemens beabstandet ist.

10. Herzfrequenzmonitor nach Anspruch 8 oder 9, wobei das Anzeigegehäuse (504) ein im Wesentlichen ebenes Display (516) hat, angeordnet in einer ersten Ebene, und das Eingabegerät (506) eine im Wesentlichen ebene Pressfläche hat, die in einer zweiten Ebene angeordnet ist, wobei die erste und zweite Ebene Winkel zueinander bilden, wobei der Winkel zwischen der ersten und zweiten Ebene weniger als 90 Grad, optional 20 bis 70 Grad beträgt.

11. Herzfrequenzmonitor nach einem der Ansprüche 8 bis 10, wobei das Anzeigegehäuse (504) durch den Verbindungsabschnitt (518) physisch mit dem Eingabegerät (506) verbunden ist, wobei der Verbindungsabschnitt entlang der Richtung vom Anzeigegehäuse zum Eingabegerät gekrümmt oder abgewinkelt ist.

12. Herzfrequenzmonitor nach Anspruch 11, wobei der Verbindungsabschnitt (518) so gekrümmt oder abgewinkelt ist, dass, wenn das Anzeigegehäuse (504) beim Gebrauch auf der Rückseite des Handgelenks eines Benutzers angeordnet ist, der Verbindungsabschnitt (518) sich krümmt oder anderweitig um das Handgelenk verläuft, so dass sich das Eingabegerät (506) auf der Seite des Handgelenks des Benutzers befindet.

13. Herzfrequenzmonitor nach einem der Ansprüche 8 bis 12, wobei der OHR-Sensor (12) eine Unterseite zum Kontaktieren des Handgelenks des Benutzers beim Gebrauch und eine gegenüberliegende Oberseite hat, die beim Gebrauch vom Handgelenk des Benutzers weg zeigt, und wobei das Eingabegerät (506) eine im Wesentlichen ebene Fläche hat, die im Wesentlichen parallel zu und über einem Abschnitt der Unterseite angeordnet ist, wobei das Eingabegerät (506) so konfiguriert ist, dass es betätigt wird, indem es in einer Richtung, die im Wesentlichen lotrecht zu seiner im Wesentlichen ebenen Fläche ist, in einer Richtung von der Oberseite in Richtung Unterseite gepresst wird.

14. Herzfrequenzmonitor nach einem der Ansprüche 8 bis 13, wobei der Riemen (2) wenigstens zwei Öffnungen (6, 8) aufweist und das Anzeigegehäuse (504) und das Eingabegerät (506) jeweils durch eine jeweilige Öffnung in dem Riemen vorstehen.

## Revendications

1. Moniteur de fréquence cardiaque, comprenant :
un capteur de fréquence cardiaque optique (OHR) (12) ; et
une sangle (2) pour monter le capteur sur un membre d'un utilisateur,
dans lequel le capteur OHR (12) comprend une unité détectrice (22) incluant au moins un émetteur de lumière (24a, 24b) agencé de façon à émettre de la lumière dans la peau d'un utilisateur, et un photodétecteur (26) agencé de façon à capter la lumière réfléchie à travers la peau de l'utilisateur, l'unité détectrice (22) faisant saillie à partir d'une surface inférieure du capteur OHR (12), et l'unité détectrice (22) étant agencée substantiellement au centre dans la sangle (2), et
dans lequel la sangle (2) comprend des portions de bord compressibles (10a, 10b) qui se trouvent le long de côtés opposés de l'unité détectrice (22), dans lequel chaque portion de bord compressible (10a, 10b) est configurée de façon à former une bride laquelle s'étend par-dessus un bord du capteur OHR (12), et dans lequel la bride de chaque portion de bord est configurée de telle sorte que, lorsque la sangle est montée sur un membre d'un utilisateur, la bride de chaque portion de bord sera comprimée contre la peau afin d'empêcher ou de réduire la pénétration de lumière ambiante jusqu'à l'unité détectrice.

2. Moniteur de fréquence cardiaque de la revendication 1, dans lequel chaque portion de bord compressible (10a, 10b) possède, dans son état non compressé, une profondeur égale ou inférieure à celle de l'unité détectrice (22) faisant saillie de la surface inférieure du capteur OHR (12).

3. Moniteur de fréquence cardiaque de la revendication 1 ou 2, dans lequel : (i) les portions de bord compressibles (10a, 10b) sont réalisées en une première matière et le restant de la sangle est réalisé à partir d'une ou plusieurs deuxièmes matières ; ou (ii) les portions de bord compressibles (10a, 10b) et le restant de la sangle sont réalisés à partir de la même première matière, et dans lequel la première matière comprend un thermoplastique et/ou un élastomère.

4. Moniteur de fréquence cardiaque de la revendication 3, dans lequel la première matière est un vulcanisat de silicone thermoplastique.

5. Moniteur de fréquence cardiaque de la revendication 3 ou 4, dans lequel la première matière est configurée de façon à avoir une compression réglée à 23° C, dans la gamme de 20 % - 40 %.

6. Moniteur de fréquence cardiaque de n'importe quelle revendication précédente, dans lequel l'unité détectrice (22) comprend une paire de diodes électroluminescentes (24a, 24b), le photodétecteur (26) étant agencé dans un emplacement entre la paire de diodes électroluminescentes.

7. Moniteur de fréquence cardiaque de n'importe quelle revendication précédente, dans lequel la sangle sert de support, de manière détachable, au capteur OHR (12).

8. Moniteur de fréquence cardiaque de n'importe quelle revendication précédente, dans lequel le capteur OHR (12) comprend un boîtier d'afficheur (504) qui abrite un affichage (516) pour afficher des informations pour un utilisateur, et un dispositif d'entrée (506) pour naviguer à travers un menu affiché sur l'affichage (516) afin de commander le fonctionnement du capteur OHR (12), dans lequel le dispositif d'entrée (506) est espacé d'une certaine distance par rapport au boîtier d'afficheur (504).

9. Moniteur de fréquence cardiaque de la revendication 8, dans lequel le dispositif d'entrée (506) est espacé d'une certaine distance par rapport au boîtier d'afficheur (504) suivant un sens longitudinal de la sangle.

10. Moniteur de fréquence cardiaque de la revendication 8 ou 9, dans lequel le boîtier d'afficheur (504) a un affichage substantiellement planaire (516) agencé suivant un premier plan et le dispositif d'entrée (506) a une surface de pression substantiellement planaire agencée suivant un deuxième plan, dans lequel les premier et deuxième plans se situent à des angles l'un par rapport à l'autre, dans lequel l'angle entre les premier et deuxième plans est inférieur à 90 degrés, facultativement entre 20 et 70 degrés.

11. Moniteur de fréquence cardiaque de l'une quelconque des revendications 8 à 10, dans lequel le boîtier d'afficheur (504) est raccordé physiquement au dispositif d'entrée (506) par une portion de raccordement (518), la portion de raccordement étant incurvée ou inclinée le long du sens allant du boîtier d'afficheur jusqu'au dispositif d'entrée.

12. Moniteur de fréquence cardiaque de la revendication 11, dans lequel la portion de raccordement (518) est incurvée ou inclinée de telle sorte que, lorsque le boîtier d'afficheur (504) est agencé sur la partie arrière du poignet d'un utilisateur, en utilisation, la portion de raccordement (518) s'incurve ou s'étend autrement autour du poignet de telle sorte que le dispositif d'entrée (506) soit localisé sur le côté du poignet de l'utilisateur.

13. Moniteur de fréquence cardiaque de l'une quelconque des revendications 8 à 12, dans lequel le capteur OHR (12) possède une surface inférieure pour se mettre au contact du poignet de l'utilisateur en utilisation, et une surface supérieure opposée pour être dirigée dans l'autre sens du poignet de l'utilisateur en utilisation, et dans lequel le dispositif d'entrée (506) possède une surface substantiellement planaire agencée de façon à être substantiellement parallèle à, et au-dessus d'une portion de la surface inférieure, le dispositif d'entrée (506) étant configuré de façon à être opéré grâce au fait qu'il est pressé suivant un sens qui est substantiellement perpendiculaire à sa surface substantiellement planaire, suivant un sens allant de la surface supérieure jusqu'à la surface inférieure.

14. Moniteur de fréquence cardiaque de l'une quelconque des revendications 8 à 13, dans lequel la sangle (2) comprend au moins deux ouvertures (6, 8), et le boîtier d'afficheur (504) et le dispositif d'entrée (506) font chacun saillie à travers une ouverture respective dans la sangle.
